(19) **Europäisches Patentamt / European Patent Office / Office européen des brevets**

(11) **EP 3 801 761 B1**

(12) **FASCICULE DE BREVET EUROPEEN**

(45) Date de publication et mention de la délivrance du brevet:
**06.07.2022 Bulletin 2022/27**

(21) Numéro de dépôt: **19725996.3**

(22) Date de dépôt: **24.05.2019**

(51) Classification Internationale des Brevets (IPC):
**A61N 7/00** (2006.01)     **A61B 17/225** (2006.01)

(52) Classification Coopérative des Brevets (CPC):
**A61N 7/00; A61B 17/225; A61B 17/2258;**
A61N 2007/0039; A61N 2007/0082;
A61N 2007/0086

(86) Numéro de dépôt international:
**PCT/EP2019/063455**

(87) Numéro de publication internationale:
**WO 2019/224350 (28.11.2019 Gazette 2019/48)**

(54) **APPAREIL DE TRAITEMENT PAR ULTRASONS COMPORTANT DES MOYENS D'IMAGERIE DES BULLES DE CAVITATION**

ULTRASCHALLVERARBEITUNGSVORRICHTUNG MIT MITTELN ZUR ABBILDUNG VON KAVITATIONSBLASEN

ULTRASONIC PROCESSING APPARATUS COMPRISING MEANS FOR IMAGING CAVITATION BUBBLES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **25.05.2018 FR 1854428**

(43) Date de publication de la demande:
**14.04.2021 Bulletin 2021/15**

(73) Titulaires:
• **Cardiawave SA**
  **75017 Paris (FR)**
• **Institut National de la Santé et de la Recherche Médicale**
  **75013 Paris (FR)**
• **Centre national de la recherche scientifique**
  **75016 Paris (FR)**
• **Ecole Supérieure de Physique et de Chimie Industrielles de la Ville de Paris**
  **75005 Paris (FR)**

(72) Inventeurs:
• **PERNOT, Mathieu**
  **75012 PARIS (FR)**
• **SUAREZ, Daniel**
  **75017 PARIS (FR)**

(74) Mandataire: **Marks & Clerk France**
**Immeuble "Visium"**
**22, avenue Aristide Briand**
**94117 Arcueil Cedex (FR)**

(56) Documents cités:
**WO-A1-2016/156989     WO-A1-2017/182655**
**US-A1- 2011 054 315**

• **B Arnal, J Baranger, C Demene, M Tanter and M Pernot: "In vivo real-time cavitation imaging in moving organs"; "3" In: "Physics in Medicine & Biology", 10 janvier 2017 (2017-01-10), IOP Publishing, XP002788998, vol. 62, pages 843-857, le document en entier**

**Description**

L'invention porte sur un appareil de traitement par ultrasons, du corps humain ou animal. Elle s'applique en particulier au traitement par ultrasons focalisés de haute intensité (HIFU, de l'anglais « High Intensity Focused Ultrasounds »), histotripsie, lithotripsie, thrombotripsie etc. d'organes mouvants, c'est-à-dire typiquement les organes abdominaux et thoraciques.

L'histotripsie est une technique de fragmentation mécanique des tissus au moyen d'impulsions ultrasonores focalisées qui génèrent des nuages de bulles de cavitation (appelé aussi « nuage de cavitation » dans un souci de simplicité). L'utilisation de l'histotripsie pour traiter diverses pathologies est une approche prometteuse pour remplacer les chirurgies à haut risque. La cavitation permet de détruire mécaniquement les tissus indésirables dans une zone focale très contrôlée, de manière non invasive et sans effets thermiques. Cependant, le corps humain est un milieu très hétérogène et le fait que la thérapie ait lieu à l'intérieur du corps sans aucun retour visuel direct soulève le besoin crucial de surveiller précisément la zone de cavitation. En particulier, pour les applications cardiaques, la cage thoracique peut provoquer des aberrations importantes sur le trajet ultrasonique de thérapie.

Des considérations similaires s'appliquent à des techniques conceptuellement proches de l'histotripsie, telles que la lithotripsie (fragmentation de calculs) et la thrombotripsie (fragmentation de caillots sanguins), ou encore le domaine de la thérapie par ultrasons focalisés de haute intensité.

L'analyse des harmoniques de la fréquence des échos ultrasonores rétrodiffusés permet de détecter la cavitation de manière passive, mais pas de localiser précisément le nuage de bulles.

Il est possible d'utiliser des techniques d'imagerie ultrasonore classique, en mode B, qui permettent une visualisation en temps réel du phénomène, ainsi que des structures anatomiques qui sont traitées. Cette approche n'est cependant pas pleinement satisfaisante. En effet, les bulles étant générées au sein de tissus biologiques hétérogènes, leurs échos peuvent être difficiles à détecter parmi l'ensemble des échos du tissu. L'identification des bulles reste donc très subjective, non quantitative, et la définition des contours du nuage de cavitation est problématique.

L'article [1] enseigne qu'une meilleure discrimination des bulles de cavitation peut être obtenue en ayant recours à une technique d'imagerie ultrasonore parallèle (ou « ultra-rapide ») associée à un filtrage spatio-temporel. Cependant, cette approche s'est avérée peu adaptée au cas où les tissus traités sont constamment en mouvement, comme par exemple dans le cas du coeur, le foie ou le rein. En effet, les ondes ultrasonores utilisées pour l'imagerie insonifient l'ensemble la région d'intérêt (voire au-delà), et leurs signaux d'écho sont très sensibles à toutes les variations spatio-temporelles qui se produisent dans cette région, et pas uniquement à celles dues à la cavitation. Cela est acceptable lorsque les tissus sont statiques ou quasi-statiques mais, en présence de tissus en mouvement, le filtrage spatio-temporel devient beaucoup moins efficace pour identifier les signaux d'échos provenant des bulles de cavitation. Or, c'est précisément dans ce dernier cas qu'il est particulièrement important de visualiser précisément le nuage de cavitation et son positionnement par rapport aux tissus.

Des méthodes de détection passive ont également été utilisées pour estimer la position des bulles de cavitation. Par exemple :

- [2] décrit une méthode de cartographie acoustique passive des bulles générées par un transducteur de thérapie d'ablation thermique. Cette méthode permet de cartographier les bulles produites par des émissions continues ou de train d'ondes de longue durée (plusieurs centaines d'oscillations). A cause de la durée des trains d'ondes il n'est pas possible d'identifier précisément la position du nuage de bulles. [2] propose une méthode pour calculer une carte d'énergie rétrodiffusée avec une résolution équivalente à la longueur d'onde utilisée, mais cela ne permet pas d'identifier précisément les frontières du nuage.
- [3] décrit une méthode alternative à [2] pour résoudre le même problème de monitoring d'émission continues.
- [4] décrit l'application de la méthode [2] pour l'histotripsie sans modification particulières.
- [5] décrit une méthode exploitant un retournement temporel des impulsions ultrasonores. Comme dans les références [2], [3] et [4], cette méthode utilise des impulsions longues et ne permet pas d'identifier précisément les frontières du nuage de bulles de cavitation.

L'invention vise à surmonter les inconvénients précités de l'art antérieur, et plus particulièrement à procurer une technique d'imagerie permettant une localisation et une segmentation précise du nuage de cavitation, y compris lors du traitement de tissus mouvants.

Conformément à l'invention, ce but est atteint par l'association de l'imagerie ultrasonore active des tissus, de l'imagerie de cavitation passive synchrone et d'un filtrage spatio-temporel (par exemple, la décomposition en valeurs singulières). L'imagerie passive synchrone se base sur la reconstruction des échos des interactions du faisceau ultrasonore thérapeutique avec le milieu, cadencée par la séquence d'émission des pulses thérapeutiques. Elle est robuste au mouvement des tissus et de bonnes performances peuvent être obtenues également là où le tissu est en mouvement.

La technique de l'invention permet de localiser le nuage de bulles de cavitation avec une résolution spatiale autant meilleure que la durée des impulsions ultrasonores utilisées pour l'imagerie, c'est-à-dire les impulsions ultrasonores de thérapie, est brève. Ainsi, de préférence, ces impulsions présenteront une durée comprise entre 0,1 μs et 50 μs (ce qui, de toute façon, est souhaitable pour des impulsions thérapeutiques focalisées). Les méthodes d'imagerie décrites dans les références [2] à [5] ne tireraient aucun bénéfice de l'utilisation d'impulsions brèves.

L'application d'un filtrage spatio-temporel à l'imagerie passive de cavitation a déjà été suggérée par le document [6], mais dans un contexte complètement différent, celui de l'ouverture de barrière hémato-encéphalique par ultrasons. Cette technique utilise des microbulles injectées et des ultrasons d'intensité insuffisante pour engendrer des bulles de cavitation. L'acquisition des échos se fait par ailleurs à travers le crâne, ce qui rend pratiquement impossible le recours à des techniques ultrasonores actives pour imager les tissus traités.

Un objet de l'invention est donc un appareil de traitement par ultrasons comprenant :

- un transducteur ultrasonore de thérapie, adapté pour générer des ondes ultrasonores focalisées ;
- un transducteur ultrasonore d'imagerie associé au transducteur ultrasonore de thérapie ; et
- un système électronique configuré pour :

    - piloter le transducteur ultrasonore de thérapie de manière à émettre un train d'impulsions d'ondes ultrasonores d'énergie et de durée adaptées pour provoquer la génération d'un nuage de bulles de cavitation dans la tache focale du transducteur lorsque ladite tache focale est positionnée à l'intérieur d'une région à traiter d'un corps humain ou animal ;
    - piloter le transducteur ultrasonore d'imagerie de manière à émettre, entre deux impulsions d'ondes ultrasonores émises par le transducteur ultrasonore de thérapie, des ondes ultrasonores dirigées vers la région à traiter, acquérir des échos des dites ondes ultrasonores et les traiter pour reconstruire au moins une image de la région à traiter ;
    - acquérir une pluralité de signaux d'échos de N>1 impulsions d'ondes ultrasonores émises par le transducteur ultrasonore de thérapie captés par le transducteur ultrasonore d'imagerie ;
    - traiter les signaux acquis par un algorithme de formation de faisceau, de manière à former des images d'échos respectives, et par un filtrage spatio-temporel permettant d'extraire des composantes desdites images d'échos représentatives d'une rétrodiffusion des impulsions d'ondes ultrasonores par les bulles de cavitation, les séparant de composantes représentatives d'une rétrodiffusion par des tissus de la région à traiter, de manière à reconstruire une image du nuage de bulles de cavitation (ces opérations pouvant être effectuées dans cet ordre ou dans l'ordre inverse) ; et
    - afficher ladite image du nuage de bulles de cavitation superposée à ladite image de la région à traiter.

Selon des modes de réalisation particuliers d'un tel appareil :

- Ledit système électronique peut être également configuré pour, lors de la reconstruction de l'image du nuage de bulles de cavitation, introduire une compensation d'une différence de temps de propagation des ondes ultrasonores entre le transducteur ultrasonore de thérapie et la tache focale, et entre la tache focale et le transducteur ultrasonore d'imagerie.
- Ledit système électronique peut être également configuré pour piloter le transducteur ultrasonore de thérapie de manière à émettre un train d'impulsions d'ondes ultrasonores de durée comprise entre 0,1 μs et 50 μs et de préférence entre 0,5 μs et 20 μs.
- N peut être compris entre 2 et 10.000 et de préférence entre 2 et 1.000.
- Ledit système électronique peut être également configuré pour reconstruire l'image du nuage de bulles de cavitation au moyen d'un algorithme de formation de faisceau parallèle.
- Ledit système électronique et le transducteur ultrasonore de thérapie peuvent être configurés pour émettre des impulsions de durée comprise entre 0,1 μs et 50 μs d'ondes ultrasonores a une fréquence centrale comprise entre 100 KHz et 5 MHz, à une cadence comprise entre 1 et 1000 Hz.
- Ledit système électronique et le transducteur ultrasonore d'imagerie peuvent être configurés pour émettre des impulsions d'ondes ultrasonores ayant une fréquence centrale compris entre 2 et 15 MHz.
- Ledit système électronique peut être également configuré pour effectuer ledit filtrage spatio-temporel par décomposition en valeurs singulières.
- Le transducteur ultrasonore de thérapie et le transducteur ultrasonore d'imagerie peuvent être agencés de manière coaxiale.
- L'appareil peut comprendre également de moyens pour déplacer la tâche focale des impulsions d'ondes ultrasonores émises par le transducteur ultrasonore de thérapie dans la région de traitement.
- Ledit système électronique peut être également configuré pour ajuster un niveau de puissance des impulsions d'ondes ultrasonores émises par le transducteur ultrasonore de thérapie en fonction de l'image du nuage de bulles de cavitation.

D'autres caractéristiques, détails et avantages de l'invention ressortiront à la lecture de la description faite en référence aux dessins annexés donnés à titre d'exemple et qui représentent, respectivement :

- La figure 1, un appareil selon un mode de réalisation de l'invention ;
- La figure 2, un schéma fonctionnel d'un tel appareil ;
- La figure 3, une séquence d'imagerie passive selon un mode de réalisation de l'invention ;
- La figure 4, une illustration de l'algorithme de décomposition en valeurs singulières ;
- La figure 5, une image de cavitation obtenue par un appareil selon un mode de réalisation de l'invention ; et
- La figure 6, un graphique illustrant un effet technique de l'invention.

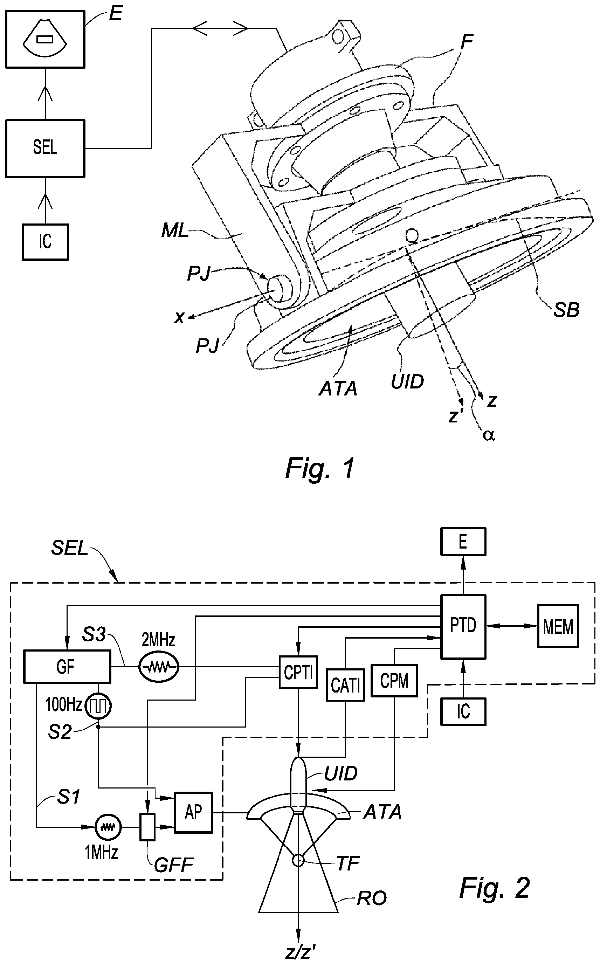

L'appareil de la figure 1 comprend un transducteur ultrasonore d'imagerie UID agencé au centre d'un transducteur de thérapie ATA constitué par un ensemble de transducteurs élémentaires de forme annulaire, de préférence en nombre compris entre 5 et 20, agencés de manière concentrique et alignés sur un bol sphérique SB qui assure une focalisation des ondes ultrasonores. Le transducteur d'imagerie UID se trouve au centre de l'anneau le plus interne du transducteur de thérapie.

Le transducteur UID comprend une pluralité d'éléments détecteurs d'ultrasons agencés en un réseau monodimensionnel (typiquement linéaire) ou bidimensionnelle, périodique ou non périodique. Il peut par exemple s'agir d'une sonde échographique bidimensionnelle comprenant un réseau de 64 éléments,

Le transducteur d'imagerie UID est fixé à un bâti F lui permettant d'être relié à un bras mécanique (non représentée) relié au transducteur de thérapie par une liaison mécanique ML permettant un mouvement de rotation relative autour de l'axe Ox (la référence PJ représente un joint pivot). Ainsi il est possible de maintenir une orientation fixe du transducteur d'imagerie (selon l'axe Oz) tout en modifiant - au moyen d'un moteur électrique non représenté - celle du transducteur de thérapie (axe Oz') de manière à permettre un déplacement de la tache focale de ce dernier perpendiculairement à l'axe Oz. En outre, en contrôlant finement le décalage entre les signaux de pilotage des différents transducteurs élémentaires il est possible de modifier la longueur focale du transducteur de thérapie, et donc la position de la tache focale le long de l'axe Oz'. Cela permet un balayage bidimensionnel d'une région à traiter par des moyens hybrides mécaniques et électroniques.

Des variantes sont possibles ; par exemple la liaison mécanique peut également permettre une rotation du transducteur de thérapie selon un deuxième axe Oy, perpendiculaire tant à Ox qu'à Oz.

L'ensemble de la figure 1 est décrit plus en détail dans la demande de brevet européen EP 3 236 467 A1. Il est donné uniquement à titre d'exemple, car de nombreuses variantes permettent la mise en oeuvre de l'invention. Par exemple, le balayage de la région à traiter par la tache focale du transducteur de thérapie peut être assuré par des moyens purement mécaniques, ou inversement purement électroniques grâce à l'utilisation d'une matrice de transducteurs élémentaires. Il n'est d'ailleurs pas essentiel que le transducteur d'imagerie soit coaxial au transducteur de thérapie, ni même relié mécaniquement à ce dernier : il suffit que leur positionnement relatif soit contrôlé et que la tache focale des impulsions ultrasonores de thérapie se trouve à l'intérieur d'une région d'observation du transducteur d'imagerie.

Par ailleurs, le transducteur d'imagerie peut présenter un nombre différent d'éléments, ou être de type bi-plan ou matriciel.

Dans tous les cas, un système électronique SEL doit être prévu pour piloter le transducteur d'imagerie et de thérapie de manière à :

- générer des impulsions d'ondes ultrasonores focalisées, adaptées pour induire la formation d'un nuage de bulles de cavitation ;
- déplacer la tâche focale de ces ondes ultrasonores de manière à balayer une région à traiter ;
- acquérir et afficher des images échographiques de la région à traiter et du nuage de bulles de cavitation, et les afficher sur un écran E.

Le système électronique SEL comprend à la fois un ou plusieurs processeurs exécutant des programmes stockés dans une mémoire et des circuits électroniques analogiques et/ou numériques opérant sous le contrôle de ce ou ces processeurs. La figure 2 en montre un schéma fonctionnel. Sur ce schéma, la référence PTD désigne un processeur de traitement de données ; d'une manière générale il peut s'agir d'un caret à microprocesseur ou microcontrôleur, d'un ordinateur ou d'un ensemble plus complexe de circuits électroniques numériques programmables. Ce processeur de données reçoit des commandes et/ou des paramètres de la part d'un utilisateur, ou opérateur, par l'intermédiaire d'un dispositif d'interface IC (clavier, souris d'ordinateur...) ; contrôle le fonctionnement d'un générateur de fonctions GF, d'un circuit de pilotage du transducteur d'imagerie CPTI, d'un circuit de formation de faisceau GFF et un circuit de pilotage d'un moteur électrique CPM ; et traite les signaux issus d'un circuit d'acquisition du transducteur d'imagerie pour reconstruire des images affichées sur l'écran E.

Le générateur de fonctions GF génère une pluralité de signaux électroniques qui sont fournis à d'autres blocs fonctionnels du système électronique. Un premier signal S1, par exemple sinusoïdal à une fréquence de 1 MHz, est fourni au circuit formateur de faisceau GFF qui le décompose en une pluralité de signaux individuels de même fréquence présentant des déphasages différents ; ces différents signaux sont amplifiés par l'amplificateur de puissance AP avant d'être appliqués aux transducteurs élémentaires du transducteur d'imagerie. Comme expliqué en détail dans la demande de brevet européen EP 3 236 467 A1 précitée, les déphasages introduits par le circuit formateur de faisceau GFF permettent de varier la longueur focale du transducteur de thérapie, et donc la position de la tache focale des ultrasons TF le long de l'axe Oz'. Le générateur de fonction génère aussi un signal rectangulaire S2 de fréquence beaucoup plus basse, par exemple 100 Hz, qui active et désactive l'amplificateur de puissance ; de cette façon le transducteur de thérapie émet des impulsions ultrasonores (« impulsions de thérapie ») à une cadence de 100 Hz, ayant par exemple une durée de 8 μs.

Le générateur de fonctions GF génère également un troisième signal S3 à haute fréquence, par exemple 2 MHz, qui est fourni avec le signal S2 au circuit de pilotage du transducteur d'imagerie CPTI. Ce dernier pilote le transducteur d'imagerie pour émettre des impulsions ultrasonores de faible intensité dans les intervalles entre les impulsions plus intenses émises par le transducteur de thérapie. La propagation de ces impulsions définit une région d'observation RO qui, dans le cas d'un transducteur d'imagerie bidimensionnel, présente une forme de trapèze dont la hauteur coïncide avec l'axe Oz.

Le circuit d'acquisition du transducteur d'imagerie CATI acquiert des signaux d'écho détectés par le transducteur d'imagerie UID, les convertit au format numérique et les transmets au processeur PTD qui procède à leur traitement. Dans une première fenêtre temporelle qui suit l'émission des impulsions ultrasonores de thérapie et dont la durée (par exemple 250 μs) dépend de la profondeur maximale de la tache focale TF, le transducteur d'imagerie détecte les échos des impulsions de thérapie, et fonctionne donc en mode passif ; comme cela sera expliqué en détail dans la suite, ces signaux d'écho permettent au processeur PTD de reconstruire des images du nuages de bulles de cavitation. Dans une deuxième fenêtre temporelle, qui s'étend de la fin de la première fenêtre jusqu'à l'émission de l'impulsion de thérapie suivante, le transducteur d'imagerie détecte les échos des impulsions qu'il a lui-même émis ; cela permet au processeur PTD de reconstruire des images des structures anatomiques de la région à traiter par des techniques conventionnelles d'imagerie ultrasonore active.

Dans le cas des impulsions utilisées pour l'imagerie ultrasonore active, le même transducteur sert de source et de détecteur d'ultrasons. Tel n'est pas le cas en imagerie passive, où les impulsions ultrasonores sont émises par le transducteur de thérapie et leurs échos sont détectés par le transducteur d'imagerie. Pour pouvoir reconstruire les images du nuage de bulles de cavitation, acquises en mode passif, il est donc nécessaire de connaitre la différence entre les temps de parcours « aller » (du transducteur de thérapie à la tache focale) et « retour » (de la tache focale au transducteur d'imagerie). Cela peut être obtenu par calcul, si la configuration mécanique de l'appareil est connue avec une précision suffisante, mais on préfèrera en règle générale procéder à un étalonnage. Pour cela, on peut procéder de plusieurs façons différentes.

- Premièrement, il est possible de placer un hydrophone en correspondance de la tache focale et mesurer les instants d'arrivée des impulsions émises par les deux transducteurs.
- Deuxièmement, il est possible de placer un réflecteur en correspondance de la tache focale et utiliser le transducteur d'imagerie en réception pour mesurer les instants d'arrivée des échos d'impulsions émises par le transducteur d'imagerie lui-même et par le transducteur de thérapie.

Des approches hybrides (utilisation de l'hydrophone pour mesurer le temps de parcours « aller », du réflecteur pour le temps de parcours « retour ») sont également possibles.

Quelle que soit la méthode utilisée, il est nécessaire de calculer ou mesurer la différence de temps de parcours en correspondance d'une pluralité de points échantillonnant toute la région à traiter.

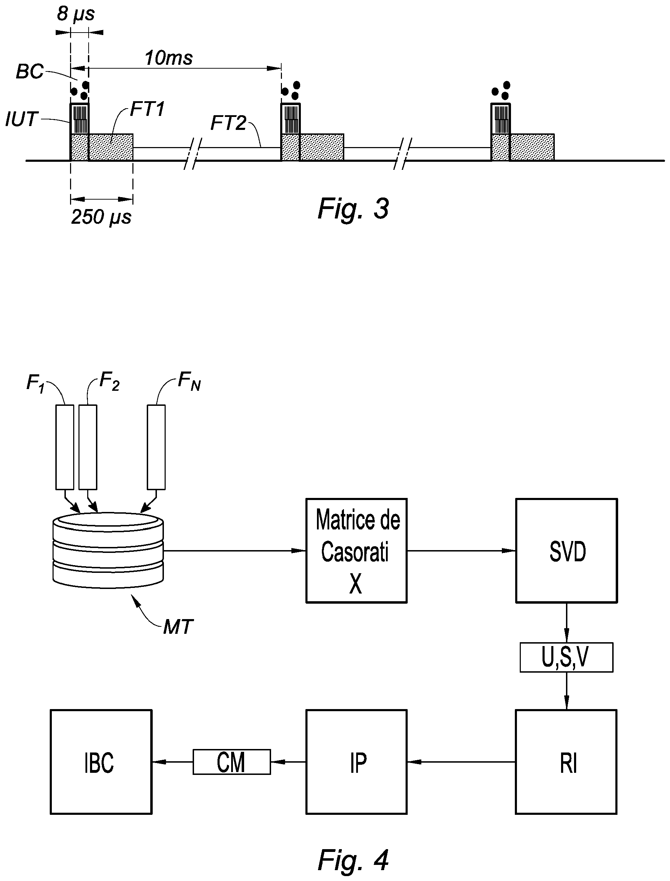

La figure 3 est un diagramme temporel montrant les impulsions de thérapie IUT d'une durée de 8 μs, émise à une cadence de 100 Hz (périodicité de 10 ms), qui engendrent un nuage de bulles de cavitation BC ; les premières fenêtres temporelles FT1 d'acquisition d'échos, qui s'étendent pendant 250 μs depuis le début de chaque impulsion de thérapie et les deuxièmes fenêtres temporelles FT2 d'imagerie active qui démarrent à la fin de chaque première fenêtre jusqu'à l'émission de l'impulsion de thérapie suivante.

Le circuit de pilotage de moteur CPM actionne un moteur électrique permettant le pivotement du transducteur de thérapie ; il coopère avec le circuit de formation de faisceau GFF pour déplacer la tache focale des impulsions ultrasonores TF afin de balayer la région à traiter. Il peut être absent des modes de réalisations dans lesquels le déplacement de la tache focale est assuré par des moyens purement électroniques (réciproquement, dans d'autres modes de réalisation le circuit de formation de faisceau peut être omis).

Les différents blocs fonctionnels décrits ci-dessus (GF, CPTI, CATI, CPM, GFF, AP) ne correspondent pas nécessairement à des composants physiquement distincts. Par exemple, un seul circuit intégré ou carte électronique peut réaliser, en tout ou en partie, les fonctions de plusieurs de ces blocs. Réciproquement, les fonctions d'un seul bloc peuvent être réalisées par plusieurs circuits intégrés et/ou cartes électroniques.

Les caractéristiques des impulsions ont été données à titre d'exemple non limitatifs. Plus généralement, le transducteur d'imagerie peut émettre des trains d'impulsions de durée comprise typiquement entre 0,1 $\mu$s et 50 $\mu$s (préférentiellement entre 0,5 $\mu$s et 20 $\mu$s) à une fréquence centrale comprise entre 100 kHz et 5 MHz et à une cadence comprise entre 1 et 1000 Hz, adaptées pour générer au point focal une pression positive de pic comprise entre 50 MPa et 100 MPa et une pression négative de pic comprise entre -2,5 MPa et -30 MPa. Le transducteur d'imagerie fonctionne généralement à une fréquence supérieure à celle du transducteur de thérapie, typiquement comprise entre 2 et 15 MHz ; par exemple, il peut émettre des ondes ultrasonores à 2 MHz et, en réception, présenter une fréquence d'échantillonnage de 8 MHz.

Comme indiqué plus haut, un appareil selon l'invention acquiert des images échographiques à la fois en mode actif, pour visualiser les tissus de la région à traiter, et en mode passif, pour visualiser le nuage de bulles de cavitation. Les images sont ensuite fusionnées afin d'être affichées sur un écran E, ce qui permet un contrôle visuel de la position du nuage de cavitation par rapport aux tissus. De préférence, ces traitements s'effectuent en temps réel. Les signaux acquis en mode actif permettent également d'estimer l'énergie absorbée par la cavitation, et d'évaluer donc l'efficacité (et/ou les dangers) du traitement en cours.

Le processeur PTD peut ainsi contrôler automatiquement l'intensité de l'impulsion de thérapie, et/ou arrêter le traitement en cas de danger. Par exemple, le processeur peut augmenter progressivement l'intensité des impulsions ultrasonores jusqu'à détecter un nuage de cavitation ayant les propriétés (forme, dimensions, énergie...) voulues ou, inversement, diminuer l'intensité tout en s'assurant de la persistance de la cavitation. Le processeur peut également arrêter le traitement lorsqu'une dose voulue a été déposée, ou si le nuage de cavitation n'est pas détecté à l'endroit voulu et risque donc d'endommager des tissus ne devant pas être traités.

Le traitement des signaux d'imagerie active peut être parfaitement conventionnel, et ne nécessite donc d'être décrit plus en détail. En revanche, afin de pouvoir extraire efficacement les images des bulles de cavitation, le processeur PTD doit soumettre les signaux acquis de manière passive à un traitement spécifique. Ce traitement comprend :

- l'acquisition des signaux d'écho de N impulsions de thérapie, généralement successives, N étant typiquement compris entre 2 et 10.000 et de préférence entre 2 et 1.000 ;
- l'application à ces signaux d'écho d'un algorithme de reconstruction par formation de faisceau (« beamforming » en anglais) pour reconstituer N images ; et
- le filtrage spatio-temporel des images ainsi reconstruites pour en extraire la contribution due aux bulles de cavitation.

La valeur de N doit être strictement supérieure à 1 pour permettre le filtrage temporel des signaux d'écho. La limite supérieure admissible dépend de la fréquence de répétition des impulsions ultrasonores ; avec une cadence élevée, de 1.000 impulsions par seconde, et N=10.000 on obtient une image filtrée toutes les 10 secondes, ce qui parait la cadence minimale acceptable pour la plupart des applications chirurgicales.

La reconstruction d'image se fait de préférence par un algorithme de formation de faisceau (à ne pas confondre avec la formation de faisceau des impulsions de thérapie) de type parallèle - ou ultrarapide - connu en soi, modifié cependant pour y inclure une compensation de la différence de temps de propagation dans les trajets aller et retour (discutée plus haut). Cet algorithme consiste essentiellement en une somme cohérente des signaux détectés par les différents éléments de la sonde d'imagerie, ces signaux étant décalés temporellement de manière à compenser leur délai de propagation des ondes ultrasonores dans les trajets aller et retour (principe de la focalisation électronique). On comprend que la prise en compte du décalage spatial entre la source des ultrasons et le détecteur est nécessaire pour une focalisation efficace. Dans le domaine spectral, la compensation du décalage est réalisée par un déphasage des signaux.

Le filtrage spatio-temporel peut utiliser tout algorithme de séparation aveugle de source permettant de différencier les échos provenant des bulles de cavitation de ceux issus, en particulier, des tissus. Il peut s'agir, par exemple, d'une décomposition en valeurs singulières, d'une analyse en composantes principales, indépendantes ou parcimonieuse, d'une factorisation en matrices non-négatives, etc. Dans la suite on considérera le cas de la décomposition en vecteurs singuliers.

La décomposition en valeurs singulières (SVD, de l'anglais « Singular Value Décomposition ») est une technique de décomposition algébrique de matrices. Elle s'adapte aux statistiques locales d'une image et concentre une énergie maximale dans un petit nombre de vecteurs propres. Elle consiste à factoriser une matrice $X_{mxn}$ dans la forme où U est une matrice orthogonale de dimensions mxm, V est une matrice orthogonale nxn et S est une matrice mxn dont les éléments diagonaux $\sigma_i$ sont les valeurs singulières de X et les autres éléments sont nuls. Si n<m on a :

$$\begin{pmatrix} X_{11} & \cdots & X_{1n} \\ \vdots & \ddots & \vdots \\ X_{m1} & \cdots & X_{nm} \end{pmatrix} = \begin{pmatrix} U_{11} & \cdots & U_{1m} \\ \vdots & \ddots & \vdots \\ U_{mm} & \cdots & U_{mm} \end{pmatrix} \cdot \begin{pmatrix} \sigma_1 & \cdots & 0 & 0\cdots0 \\ \vdots & \ddots & \vdots & \vdots \vdots \vdots \\ 0 & \cdots & \sigma_n & 0\cdots0 \end{pmatrix} \cdot \begin{pmatrix} V_{11} & \cdots & V_{1n} \\ \vdots & \ddots & \vdots \\ V_{n1} & \cdots & V_{nn} \end{pmatrix}^{*}$$

$$(Eq.\ 1)$$

où V* est la matrice conjuguée transposée de V et le nombre de valeurs singulières de S est égal au rang de X.

Lorsque la méthode SVD est appliquée au traitement d'images, les petites valeurs singulières sont associées au bruit, et la plupart de l'énergie de l'image est compactée dans les valeurs singulières de valeur élevée. Dans le cas de l'invention, le filtrage spatio-temporel SVD est utilisé pour séparer les composantes de basse fréquence, indésirables, des hautes fréquences associées au nuage de bulles de cavitation par un choix opportun des vecteurs singuliers. Cela est illustré sur la figure 4.

On considère un ensemble de N images « passives » $F_1$, $F_2$, ... $F_N$, associées à des impulsions de thérapie respectives, reconstruites par un algorithme de formation de faisceau parallèle, chaque image étant de dimensions ($n_x$, $n_y$), et stockées dans une mémoire tampon MT. Ces données peuvent être réarrangées en une matrice spatio-temporelles bidimensionnelle X de dimensions ($n_x \cdot n_y$, N), dite matrice de Casorati. La décomposition en valeurs singulière de cette matrice (référence SVD sur la figure 4) consiste à trouver des vecteurs singuliers temporels et spatiaux formant, respectivement, les colonnes des matrices U et V, ainsi que les valeurs singulières correspondantes formant la matrice S. Avantageusement ces vecteurs sont ordonnés par énergie décroissante.

Le filtrage spatio-temporel proprement dit consiste à reconstruire l'image en utilisant uniquement les vecteurs singuliers qui décrivent le nuage de bulles. On suppose que ces vecteurs sont associés à des valeurs singulières contigües d'indices compris entre p et q>p. L'image filtrée $X_{BC}$ est donc donnée par :

$$X_{BC}(n_x \times n_z, N) = \sum_{i=p}^{q} \sigma_i U_i V_i^{T}$$

$$(Eq.2)$$

(référence RI sur la figure 4).

Les termes de la matrice de Casorati filtrée $X_{BC}(n_x n_z, N)$ peuvent être réarrangés en une matrice tridimensionnelle $X_{BC}$ ($n_x$, $n_y$, N), et il est possible de calculer son intégral de puissance, ou carte de cavitation CM, qui permet de localiser les régions les plus énergétiques de l'image :

$$CM(n_x, n_z) = \int |X_{BC}(n_x, n_z, N)|^2$$

$$(Eq.3)$$

(référence IP sur la figure 4).

Il est important de noter que N images donnent une seule carte de cavitation; en d'autres termes, la cadence d'acquisition est divisée par N.

La carte de cavitation est utilisée comme image finale du nuage de bulles de cavitation, destinée à être superposée à l'image des tissus obtenue par échographie active (référence IBC sur la figure 4). Des fausses couleurs peuvent être utilisées pour représenter les valeurs de la carte CM.

La résolution spatiale de la carte CM est limitée par la longueur des impulsions ultrasonores dans la direction de propagation, donnée par le produit de leur durée par leur vitesse de propagation. Ainsi, l'on utilisera de préférence des impulsions aussi brèves que possibles, à la limite de durée égale à un cycle de l'onde ultrasonore. On entend par durée des impulsions la durée (c'est-à-dire le support temporel) des impulsions électroniques de pilotage du transducteur de thérapie, étant entendu que la largeur de bande finie de ce dernier entraine inévitablement un allongement des impulsions ultrasonores effectivement émises. En pratique, la durée des impulsions n'est pas choisie uniquement en considération de la résolution d'imagerie mais également, voire surtout, en fonction des exigences du protocole thérapeutique.

Le choix des valeurs optimales des paramètres N, p et q dépend de l'application spécifique considérée. Les inventeurs ont constaté que les signaux d'écho provenant des tissus (que l'on souhaite éliminer) se trouvent essentiellement concentrés dans le premier vecteur singulier, les N-1 vecteurs singuliers suivant contenant principalement les contributions des bulles de cavitation (que l'on souhaite isoler). On pourra donc choisir p=2 et q=N. En ce qui concerne le paramètre N, les inventeurs ont constaté que l'écart contraste à bruit (défini plus loin) croit rapidement avec N tant que la valeur de ce paramètre reste inférieure ou égale à environ 10, puis tend à plafonner. Dans la suite, trois cas ont été considérés : N=6 ; N=10 et N=14.

L'écart contraste à bruit, qui définit la qualité des images (plus précisément, des cartes de cavitation) est donné par :

$$CNR = \frac{<CM(n_x, n_z)>_1 - <CM(n_x, n_z)>_2}{\sqrt{\sigma_1 \sigma_2}}$$

$$(Eq.4)$$

où $<>_i$ désigne une moyenne spatiale et $\sigma_i$ l'écart type dans ces deux régions dans la région i (i=1 : nuage de bulles; i=2: arrière-plan). Les régions 1 et 2 ont été identifiées manuellement.

En variante, l'étape de filtrage spatio-temporel peut être mise en oeuvre avant la formation du faisceau. Dans ce cas, la composante spatiale du filtrage porte sur les éléments de la sonde d'imagerie.

Afin de tester l'invention, un fantôme a été préparé avec un volume de 1,75 L d'eau et 8% d'alcool polyvinylique (PVA). Des diffuseurs d'ultrasons ont été ajoutés en utilisant 1% de cellulose (Sigmacell, 20 $\mu$m, USA). L'eau du robinet a été chauffée à 90 ° C en utilisant un appareil de chauffage de laboratoire et le volume nécessaire a été versé dans un bêcher avec un pivot de direction magnétique. Le PVA a ensuite été dissous dans l'eau. Le mélange a été refroidi à 40 ° C, de la cellulose a été ajoutée et la solution a été versée dans un récipient carré en plastique et placée dans le congélateur pendant 8 heures. Le fantôme a ensuite été décongelé, puis remis au congélateur pendant 8 heures. À la fin de la procédure, le fantôme a été démoulé et placé dans un réservoir d'eau pour les expériences de cavitation.

L'assemblage de transducteurs d'un appareil selon l'invention était attaché à un étage à deux axes (PI, Micos, Allemagne) et disposé à l'intérieur du réservoir d'eau contenant le fantôme. Pour modéliser un mouvement physiologique de la région du corps à traiter, des mouvements axiaux et latéraux des transducteurs ont été induits avec des vitesses différentes, une vitesse de pointe de 10 mm / s avec une amplitude de mouvement de 10 mm pour le mouvement axial et une vitesse de pointe de 10 mm/ s avec une amplitude de mouvement de 8 mm pour le mouvement latéral.

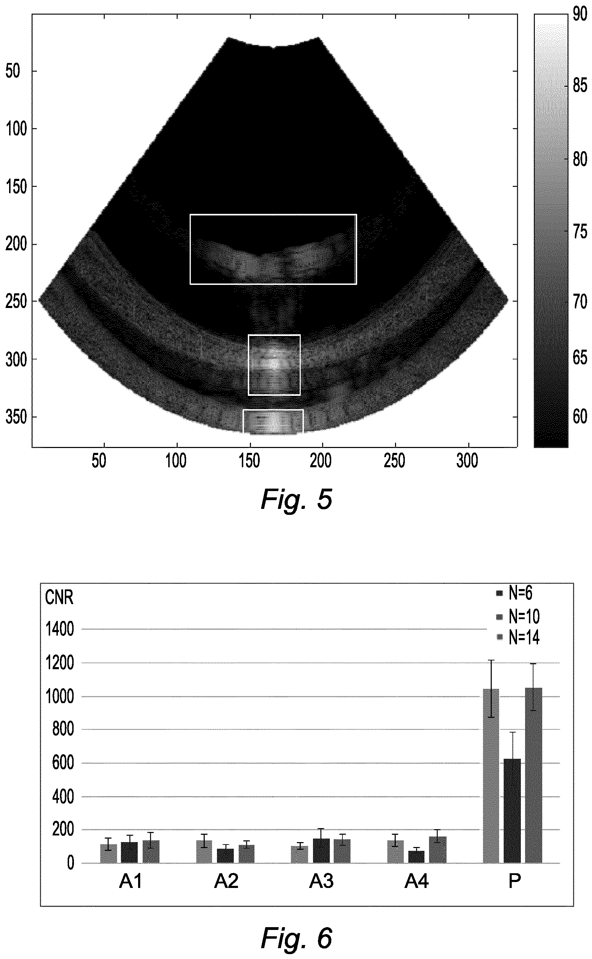

La figure 5 montre une image du nuage de bulles de cavitation obtenu de cette façon. Le nuage est identifié par un encadré blanc. Des artefacts sont présents au-dessus et au-dessous de l'image ; ils sont vraisemblablement dus aux réflexions des impulsions ultrasonores par les interfaces eau-fantôme.

L'écart contraste à bruits CNR des images « passives » du nuage de bulles de cavitation obtenues pour N=6, 10 et 14 (avec toujours p=2 et q=N) a été mesuré, aussi bien en conditions statiques qu'avec les transducteurs en mouvement. Des mesures semblables ont également été effectuées en utilisant des techniques d'imagerie active. Les inventeurs ont constaté que l'imagerie active des bulles de cavitation donne des résultats légèrement meilleurs que l'imagerie passive en conditions statiques, mais que l'inverse est vrai avec les transducteurs en mouvement. Cela valide l'utilisation d'une méthode passive dans l'invention.

Les résultats obtenus avec les transducteurs en mouvement sont illustrés par la figure 6, où la référence P désigne le CNR des images passives et A1 - A5 celui des images obtenues par des méthodes actives :

- A1 : imagerie active obtenue avec émission d'une onde plane d'ultrasons après chaque impulsion de thérapie.
- A2 : séquence ultrarapide avec un bloc de onze émissions d'ondes divergentes dirigées après chaque impulsion de thérapie, combinées en une seule image.
- A3 : séquence ultrarapide avec un bloc de dix émissions d'ondes divergentes (sans combinaison) après chaque impulsion de thérapie, ce qui donne un bloc de dix images toutes les 10 millisecondes.
- A4 : séquence ultra-rapide avec un bloc de cent dix émissions d'ondes divergentes dirigées réparties sur trois impulsions de thérapie, qui sont combinées en dix images. Chaque image est le résultat de la combinaison de onze ondes divergentes orientées. Cette séquence donne un bloc de dix images environ toutes les 35 millisecondes.

En fait, ce qu'on observe est que les mouvements des transducteurs induisent une très forte dégradation des performances de techniques d'imagerie active (un ordre de grandeur), tandis que le CNR des images obtenues conformément à l'invention ne de- décroit que d'un facteur deux environ. Cela peut être expliqué de la manière suivante. Lorsque la région à traiter (ou, de manière équivalente, les transducteurs) est en mouvement, les réflexions par les tissus contiennent des composantes spatiotemporelles incohérentes à haute fréquence qui interfèrent avec les composantes utiles issues des bulles de cavitation. Or, en imagerie active, les tissus dans l'intégralité de la région d'observation sont exposés à des ultrasons et engendrent donc des échos. Par contre, en imagerie passive, l'on utilise des ultrasons focalisés, ce qui réduit la contribution parasite provenant des tissus.

Références :

[1] B. Arnal, J. Baranger, C. Demene, M. Tanter and M. Pernot (2016). In vivo real-time cavitation imaging in moving organs. Phys Med Biol. 2017 Feb 7;62(3):843-857.

[2] Christian Coviello, Richard Kozick, James Choi, Miklós Gyôngy, Carl Jensen, Penny Probert Smith, and Constantin-C. Coussios (2015). Passive acoustic mapping utilizing optimal beamforming in ultrasound therapy monitoring. The Journal of the Acoustical Society of America 137, 2573.

[3] Kevin J. Haworth, Kenneth B. Bader, Kyle T. Rich, Christy K. Holland, Member and T. Douglas Mast (2017). Quantitative Frequency-Domain Passive Cavitation Imaging. IEEE transactions on ultrasonics, ferroelectrics, and frequency control, vol. 64, no. 1.

[4] Bader Kenneth, Haworth Kevin, D. Maxwell Adam, Holland Christy (2017). Post Hoc Analysis of Passive Cavitation Imaging for Classification of Histotripsy-Induced Liquefaction in Vitro. IEEE Transactions on Médical Imaging.

[5] P. Boulos, F. Varray, A. Poizat, J.C. Bera, C.Cachard (2015). Passive cavitation imaging using an open ultrasonic system and time reversai reconstruction. 22èmeCongrès Français de Mécanique Lyon.

**Revendications**

1. Appareil de traitement par ultrasons comprenant :

      - un transducteur ultrasonore de thérapie (ATA), adapté pour générer des ondes ultrasonores focalisées ;
      - un transducteur ultrasonore d'imagerie (UID) associé au transducteur ultrasonore de thérapie ; et
      - un système électronique configuré pour :

         - piloter le transducteur ultrasonore de thérapie de manière à émettre un train d'impulsions d'ondes ultrasonores d'énergie et de durée adaptées pour provoquer la génération d'un nuage de bulles de cavitation (BC) dans une tache focale (TF) du transducteur lorsque ladite tache focale est positionnée à l'intérieur d'une région à traiter d'un corps humain ou animal ;
         - piloter le transducteur ultrasonore d'imagerie de manière à émettre, entre deux impulsions d'ondes ultrasonores émises par le transducteur ultrasonore de thérapie, des ondes ultrasonores dirigées vers la région à traiter, acquérir des échos des dites ondes ultrasonores et les traiter pour reconstruire au moins une image de la région à traiter ;
         - acquérir une pluralité de signaux d'échos de N>1 impulsions d'ondes ultrasonores émises par le transducteur ultrasonore de thérapie captés par le transducteur ultrasonore d'imagerie,
         - traiter lesdits signaux d'échos des N impulsions d'ondes ultrasonores émises par le transducteur ultrasonore de thérapie par un algorithme de formation de faisceau, de manière à former des images d'échos respectives ($F_1$ - $F_N$), et par un filtrage spatio-temporel permettant d'extraire des composantes desdites images d'échos représentatives d'une rétrodiffusion des impulsions d'ondes ultrasonores par les bulles de cavitation, les séparant de composantes représentatives d'une rétrodiffusion par des tissus de la région à traiter, de manière à reconstruire une image (IBC) du nuage de bulles de cavitation ; et
         - afficher ladite image du nuage de bulles de cavitation superposée à ladite image de la région à traiter.

2. Appareil de traitement par ultrasons selon la revendication 1 dans lequel ledit système électronique est également configuré pour, lors de la reconstruction de l'image du nuage de bulles de cavitation, introduire une compensation d'une différence de temps de propagation des ondes ultrasonores entre le transducteur ultrasonore de thérapie et la tache focale, et entre la tache focale et le transducteur ultrasonore d'imagerie.

3. Appareil de traitement par ultrasons selon l'une des revendications précédentes dans lequel ledit système électronique est configuré pour piloter le transducteur ultrasonore de thérapie de manière à émettre un train d'impulsions d'ondes ultrasonores de durée comprise entre 0,1 μs et 50 μs et de préférence entre 0,5 μs et 20 μs.

4. Appareil de traitement par ultrasons selon l'une des revendications précédentes dans lequel N est compris entre 2 et 10.000 et de préférence entre 2 et 1.000.

5. Appareil de traitement par ultrasons selon l'une des revendications précédentes dans lequel ledit système électronique est configuré pour reconstruire l'image du nuage de bulles de cavitation au moyen d'un algorithme de formation de faisceau parallèle.

6. Appareil de traitement par ultrasons selon l'une des revendications précédentes dans lequel le système électronique et le transducteur ultrasonore de thérapie sont configurés pour émettre des impulsions de durée comprise entre 0,1 µs et 50 µs d'ondes ultrasonores a une fréquence centrale comprise entre 100 KHz et 5 MHz, à une cadence comprise entre 1 et 1.000 Hz.

7. Appareil de traitement par ultrasons selon l'une des revendications précédentes dans lequel le système électronique et le transducteur ultrasonore d'imagerie sont configurés pour émettre des impulsions d'ondes ultrasonores ayant une fréquence centrale compris entre 2 et 15 MHz.

8. Appareil de traitement par ultrasons selon l'une des revendications précédentes dans lequel le système électronique est configuré pour effectuer ledit filtrage spatio-temporel par décomposition en valeurs singulières.

9. Appareil de traitement par ultrasons selon l'une des revendications précédentes dans lequel le transducteur ultrasonore de thérapie et le transducteur ultrasonore d'imagerie sont agencés de manière coaxiale.

10. Appareil de traitement par ultrasons selon l'une des revendications précédentes comprenant également des moyens (GFF, CPM) pour déplacer la tâche focale des impulsions d'ondes ultrasonores émises par le transducteur ultrasonore de thérapie dans la région de traitement.

11. Appareil de traitement par ultrasons selon l'une des revendications précédentes dans lequel le système électronique est également configuré pour ajuster un niveau de puissance des impulsions d'ondes ultrasonores émises par le transducteur ultrasonore de thérapie en fonction de l'image du nuage de bulles de cavitation.

**Patentansprüche**

1. Ultraschallbehandlungseinrichtung, umfassend:

   - einen Therapie-Ultraschallwandler (ATA), der dazu geeignet ist, fokussierte Ultraschallwellen zu erzeugen;
   - einen Bildgebungs-Ultraschallwandler (UID), der dem Therapie-Ultraschallwandler zugeordnet ist; und
   - ein elektronisches System, das dafür konfiguriert ist,
   - den Therapie-Ultraschallwandler derart zu steuern, dass er eine Folge von Ultraschallwellenimpulsen mit einer Energie und einer Dauer ausstrahlt, die dazu geeignet sind, die Erzeugung einer Wolke von Kavitationsblasen (BC) in einem Brennfleck (TF) des Wandlers hervorzurufen, wenn der Brennfleck im Inneren einer zu behandelnden Region eines menschlichen oder tierischen Körpers positioniert ist;
   - den Bildgebungs-Ultraschallwandler derart zu steuern, dass er zwischen zwei Ultraschallwellenimpulsen, die von dem Therapie-Ultraschallwandler ausgestrahlt werden, Ultraschallwellen, die zu der zu behandelnden Region gelenkt werden, ausstrahlt, Echos der Ultraschallwellen erfasst und diese verarbeitet, um mindestens ein Bild der zu behandelnden Region zu rekonstruieren;
   - eine Vielzahl von Echosignalen von N>1 Ultraschallwellenimpulsen zu erfassen, die von dem Therapie-Ultraschallwandler ausgestrahlt werden, die von dem Bildgebungs-Ultraschallwandler empfangen werden,
   - die Echosignale der N Ultraschallimpulse, die von dem Therapie-Ultraschallwandler ausgestrahlt werden, durch einen Strahlbildungsalgorithmus, um jeweilige Echobilder ($F_1$ - $F_N$) zu bilden, und durch ein räumlich-zeitliches Filtern zu verarbeiten, wodurch Komponenten der Echobilder, die für eine Rückstreuung der Ultraschallwellenimpulse von den Kavitationsblasen repräsentativ sind, extrahiert werden können, wobei diese von Komponenten getrennt werden, die für eine Rückstreuung von Gewebe der zu behandelnden Region repräsentativ sind, um ein Bild (IBC) der Wolke von Kavitationsblasen zu rekonstruieren; und
   - das Bild der Wolke von Kavitationsblasen dem Bild der zu behandelnden Region überlagert anzuzeigen.

2. Ultraschallbehandlungseinrichtung nach Anspruch 1, wobei das elektronische System außerdem dafür konfiguriert ist, bei der Rekonstruktion des Bildes der Wolke von Kavitationsblasen eine Kompensation einer Differenz der Ausbreitungszeit der Ultraschallwellen zwischen dem Therapie-Ultraschallwandler und dem Brennfleck und zwischen dem Brennfleck und dem Bildgebungs-Ultraschallwandler einzuführen.

3.  Ultraschallbehandlungseinrichtung nach einem der vorhergehenden Ansprüche, wobei das elektronische System dafür konfiguriert ist, den Therapie-Ultraschallwandler derart zu steuern, dass er eine Ultraschallwellenimpulsfolge einer Dauer zwischen 0,1 μs und 50 μs und vorzugsweise zwischen 0,5 μs und 20 μs ausstrahlt.

4.  Ultraschallbehandlungseinrichtung nach einem der vorhergehenden Ansprüche, wobei N zwischen 2 und 10.000 und vorzugsweise zwischen 2 und 1.000 liegt.

5.  Ultraschallbehandlungseinrichtung nach einem der vorhergehenden Ansprüche, wobei das elektronische System dafür konfiguriert ist, das Bild der Wolke von Kavitationsblasen mittels eines parallelen Beamforming-Algorithmus zur parallelen Strahlbildung zu rekonstruieren.

6.  Ultraschallbehandlungseinrichtung nach einem der vorhergehenden Ansprüche, wobei das elektronische System und der Therapie-Ultraschallwandler dafür konfiguriert sind, Impulse einer Dauer zwischen 0,1 μs und 50 μs von Ultraschallwellen mit einer Mittenfrequenz zwischen 100 KHz und 5 MHz mit einer Kadenz zwischen 1 und 1.000 Hz auszustrahlen.

7.  Ultraschallbehandlungseinrichtung nach einem der vorhergehenden Ansprüche, wobei das elektronische System und der Bildgebungs-Ultraschallwandler dafür konfiguriert sind, Ultraschallwellenimpulse auszustrahlen, die eine Mittenfrequenz zwischen 2 und 15 MHz aufweisen.

8.  Ultraschallbehandlungseinrichtung nach einem der vorhergehenden Ansprüche, wobei das elektronische System dafür konfiguriert ist, das räumlich-zeitliche Filtern durch Zerlegen in Einzelwerte auszuführen.

9.  Ultraschallbehandlungseinrichtung nach einem der vorhergehenden Ansprüche, wobei der Therapie-Ultraschallwandler und der Bildgebungs-Ultraschallwandler koaxial angeordnet sind.

10. Ultraschallbehandlungseinrichtung nach einem der vorhergehenden Ansprüche, außerdem umfassend Mittel (GFF, CPM) zum Verschieben des Brennflecks von Ultraschallwellenimpulsen, die von dem Therapie-Ultraschallwandler in die Behandlungsregion ausgestrahlt werden.

11. Ultraschallbehandlungseinrichtung nach einem der vorhergehenden Ansprüche, wobei das elektronische System außerdem dafür konfiguriert ist, einen Leistungspegel der Ultraschallimpulse, die von dem Therapie-Ultraschallwandler ausgestrahlt werden, in Abhängigkeit von dem Bild der Wolke von Kavitationsblasen einzustellen.

**Claims**

1.  An ultrasonic treatment apparatus, comprising:

    - a therapy ultrasound transducer (ATA), suitable for generating focused ultrasonic waves;
    - an imaging ultrasound transducer (UID) associated with the therapy ultrasound transducer; and
    - an electronic system configured to:
    - control the therapy ultrasound transducer so as to emit a pulse train of ultrasonic waves of an energy and a duration suitable for causing the generation of a cloud of cavitation bubbles (BC) in a focal spot (TF) of the transducer when said focal spot is positioned inside a region to be treated of a human or animal body;
    - control the imaging ultrasound transducer so as to emit, between two ultrasonic-wave pulses emitted by the therapy ultrasound transducer, ultrasonic waves that are directed toward the region to be treated, to acquire echoes of said ultrasonic waves and to process them to reconstruct at least one image of the region to be treated;
    - acquire a plurality of echo signals of N>1 ultrasonic-wave pulses emitted by the therapy ultrasound transducer, which signals are captured by the imaging ultrasound transducer;
    - process said echo signals of the N ultrasonic-wave pulses emitted by the therapy ultrasound transducer using a beamforming algorithm, so as to form respective echo images ($F_1$ - $F_N$), and using spatio-temporal filtering allowing components of said echo images that are representative of a backscatter of the ultrasonic-wave pulses by the cavitation bubbles to be extracted, separating them from components representative of a backscatter by tissues of the region to be treated, so as to reconstruct an image (IBC) of the cloud of cavitation bubbles; and
    - display, superposed on said image of the region to be treated, said image of the cloud of cavitation bubbles.

2.  The ultrasonic treatment apparatus according to claim 1, wherein said electronic system is also configured to, during the reconstruction of the image of the cloud of cavitation bubbles, introduce a compensation for a difference in propagation time of the ultrasonic waves between the therapy ultrasound transducer and the focal spot, and between the focal spot and the imaging ultrasound transducer.

3.  The ultrasonic treatment apparatus according to one of the preceding claims, wherein said electronic system is configured to control the therapy ultrasound transducer so as to emit a train of ultrasonic-wave pulses with a duration comprised between 0.1 $\mu$s and 50 $\mu$s and preferably between 0.5 $\mu$s and 20 $\mu$s.

4.  The ultrasonic treatment apparatus according to one of the preceding claims, wherein N is comprised between 2 and 10,000 and preferably between 2 and 1,000.

5.  The ultrasonic treatment apparatus according to one of the preceding claims, wherein said electronic system is configured to reconstruct the image of the cloud of cavitation bubbles by means of a parallel beamforming algorithm.

6.  The ultrasonic treatment apparatus according to one of the preceding claims, wherein the electronic system and the therapy ultrasound transducer are configured to emit ultrasonic-wave pulses with a duration comprised between 0.1 $\mu$s and 50 $\mu$s, with a central frequency comprised between 100 kHz and 5 MHz, at a repetition rate comprised between 1 and 1,000 Hz.

7.  The ultrasonic treatment apparatus according to one of the preceding claims, wherein the electronic system and the imaging ultrasound transducer are configured to emit ultrasonic-wave pulses having a central frequency comprised between 2 and 15 MHz.

8.  The ultrasonic treatment apparatus according to one of the preceding claims, wherein the electronic system is configured to perform said spatio-temporal filtering using singular value decomposition.

9.  The ultrasonic treatment apparatus according to one of the preceding claims, wherein the therapy ultrasound transducer and the imaging ultrasound transducer are arranged coaxially.

10. The ultrasonic treatment apparatus according to one of the preceding claims, also comprising means (GFF, CPM) for moving the focal spot of the ultrasonic-wave pulses emitted by the therapy ultrasound transducer in the treatment region.

11. The ultrasonic treatment apparatus according to one of the preceding claims, wherein the electronic system is also configured to adjust a power level of the ultrasonic-wave pulses emitted by the therapy ultrasound transducer depending on the image of the cloud of cavitation bubbles.

**Fig. 1**

**Fig. 2**

**Fig. 3**

**Fig. 4**

Fig. 5

Fig. 6

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 3236467 A1

**Littérature non-brevet citée dans la description**

- **B. ARNAL ; J. BARANGER ; C. DEMENE ; M. TANTER ; M. PERNOT.** In vivo real-time cavitation imaging in moving organs. *Phys Med Biol.,* 07 Février 2017, vol. 62 (3), 843-857
- **CHRISTIAN COVIELLO ; RICHARD KOZICK ; JAMES CHOI ; MIKLÓS GYÔNGY ; CARL JENSEN ; PENNY PROBERT SMITH ; CONSTANTIN-C. COUSSIOS.** Passive acoustic mapping utilizing optimal beamforming in ultrasound therapy monitoring. *The Journal of the Acoustical Society of America,* 2015, vol. 137, 2573
- **KEVIN J. HAWORTH ; KENNETH B. BADER ; KYLE T. RICH ; CHRISTY K. HOLLAND ; T. DOUGLAS MAST.** Quantitative Frequency-Domain Passive Cavitation Imaging. *IEEE transactions on ultrasonics, ferroelectrics, and frequency control,* 2017, vol. 64 (1
- **BADER KENNETH ; HAWORTH KEVIN ; D. MAXWELL ADAM ; HOLLAND CHRISTY.** Post Hoc Analysis of Passive Cavitation Imaging for Classification of Histotripsy-Induced Liquefaction in Vitro. *IEEE Transactions on Médical Imaging,* 2017
- **P. BOULOS ; F. VARRAY ; A. POIZAT ; J.C. BERA ; C.CACHARD.** Passive cavitation imaging using an open ultrasonic system and time reversai reconstruction. *22èmeCongrès Français de Mécanique Lyon,* 2015